# EUROPEAN PATENT SPECIFICATION

(11) **EP 2 046 220 B1**
(45) Date of publication and mention of the grant of the patent: **15.06.2011**
(21) Application number: 07804579.6
(22) Date of filing: 09.07.2007
(51) Int. Cl.: A61B 18/08

(54) **MEDICAL DEVICE**
MEDIZINISCHE VORRICHTUNG
DISPOSITIF MÉDICAL

(30) Priority: 11.07.2006 US 484168
(43) Date of publication of application: 15.04.2009
(73) Proprietor: Rossi, Cino, 41037 Mirandola (Modena) (IT)
(72) Inventor: Rossi, Cino, 41037 Mirandola (Modena) (IT)
(74) Representative: Bergamini, Silvio
(86) International application number: PCT/IB2007/001919
(87) International publication number: WO 2008/007203

(56) References cited:
- WO-A-2005/000001
- US-A- 6 102 885
- US-A1- 2001 029 366
- US-A1- 2002 151 889
- US-A1- 2002 193 781
- US-A1- 2004 082 945
- US-A1- 2005 033 285
- US-A1- 2005 101 909
- US-A1- 2006 064 101
- US-A1- 2006 095 059

## Description

### TECHNICAL FIELD.

The invention concerns a medical device, suitable for cutting portions of human and animal tissues.

### BACKGROUND ART.

In the execution of medical therapies, it may be necessary to cut excrescences of tissue located inside cavities of a human or animal body.

To execute these cuts, currently instruments are used that are known as electrosurgical unit which includes a machine that generates electricity with variable intensity and that is connected to a long thin metallic body, defined as the electrode, which is inserted in a sheath and which has a distal extremity preformed in the shape of a rigid loop that generally has the shape of a semicircle.

The electrode reaches the cutting point inside an endocavity by means of an endoscope, both flexible and rigid, which has been positioned inside the cavity in advance.

The endoscope is introduced inside the endocavity in such a manner that the distal extremity is located near a portion of tissue to be cut, for example an excrescence, so that the electrode's loop, which is guided by the endoscope, is able to reach it with precision.

Inside the endoscope, before inserting the electrode, a flexible needle is also inserted by sliding, which is connected to a device for injecting anesthetics such as, for example, a syringe, to anesthetize the area surrounding the portion of tissue to be cut.

Therefore, overall, to remove a portion of tissue, a doctor must insert in advance an endoscope inside of an endocavity; then he must insert the needle in the endoscope through a working channel provided for in it, so that its distal extremity is located near the point in which the cut must be made.

Then, by maneuvering the needle's proximal extremity which is purposefully kept outside the endoscope, the doctor inserts the tip of the needle in the tissue in the area to be anesthetized and injects the anesthetic in it.

When the injection is completed, the doctor removes the needle from the endoscope, while maintaining the latter in its initial operating position and then inserts the electrosurgical unit's electrode, until its distal extremity protrudes from the corresponding distal extremity of the endoscope.

The electrode's rigid tip in the shape of a loop is placed near the area of the tissue to be removed, by maneuvering the electrode from outside the endoscope.

After the loop has been correctly placed in position, the electrode will be powered with electricity at a set intensity which causes it to heat up to a temperature of approximately 300 °C; by maneuvering it properly, the doctor inserts the removed, cutting off the portion of tissue that must be removed.

The doctor then modifies the electricity of the electrosurgical unit and adequately maneuvers the electrode to place its loop in contact with the cut blood vessels to coagulate and cauterize them.

According to US patent application 2002/0193781 A1 a device for interstitial delivery of thermal energy into tissue and methods of use thereof is known.

This patent application teaches a catheter device adapted for delivering thermal energy to a body tissue, having a proximal end for connecting to an energy source, a tubular body portion and a distal end adapted for penetration into body tissues.

Nevertheless, the tubular body of the catheter device has one of the extremities which is closed by a plug which holds an electrode for generating heating and, therefore, the catheter device according to this US patent application is suitable for administering only heat to the tissue, but not substances of any kind, e.g. anaesthesia. According to the further International patent application WO2005/000001 a surgical cannula for tissue retraction is also known.

This cannula is expandable both longitudinally and radially to modify its dimensions by applying a low temperature which modifies the molecular structure of the cannula.

Nevertheless, this cannula cannot be used for cutting and removing portion of tissues of a human or animal body, because the action of the temperature modifies its shape and this feature doesn't allow to wrap and maintain the cannula e.g. around a portion of tissue to be cut by using a high cutting temperature suitable to coagulate the cut contemporarily.

This state of the art presents a drawback which is the need to use, besides the endoscope, two different instruments to cut portions of tissue, that is, a needle to anesthetize and an electrode to cut the portion of tissue and coagulate and cauterize the cut blood vessels.

A second drawback is that the doctor, while using two different instruments, must perform two maneuvers in succession, in order to insert and remove firstly from the endoscope the needle used to perform anesthesia and secondly in order to insert the electrosurgical unit's electrode to perform the cut and the cauterization of the cut tissue.

This state of the art requires a noticeable amount of time to insert and remove each time two different instruments inside the endoscope.

Furthermore, there is a risk, during the maneuver to insert and remove the needle for performing the anesthesia and the subsequent insertion of the electrode, that the endoscope may be moved away from the exact position at which the cut and removal must be carried out.

Another object of the invention is to build a medical device that makes it possible to perform cuts and remove excrescences or other portions of tissue with a single instrument, thus eliminating the need to use two different instruments to perform anesthesia in advance and to subsequently cut a portion of tissues and coagulate and cauterize the cut blood vessels.

According to an aspect of the invention it is provided a medical device, suitable for cutting and/or coagulating portions of human and animal tissues as claimed in claim 1.

Therefore, the medical device for cutting portions of human and animal tissues makes it possible to inject substances, especially anesthetizing substances, and to cut portions of tissues by using a single instrument, thereby considerably reducing the operating time necessary to perform these procedures.

### BRIEF DESCRIPTION OF DRAWINGS.

Further features and advantages will become clearer from the description of one embodiment of a medical device, suitable for cutting portions of human and animal tissues, illustrated by way of non-limitative example in the attached sheets of drawings in which:
Figures from 1a to 1d are enlarged, detailed and interrupted views of a distal portion of a medical device, suitable for cutting portions of human and animal tissues, in four consecutive steps of use;
Figure 2 is an overall view of a medical device, suitable for cutting portions of human and animal tissues;

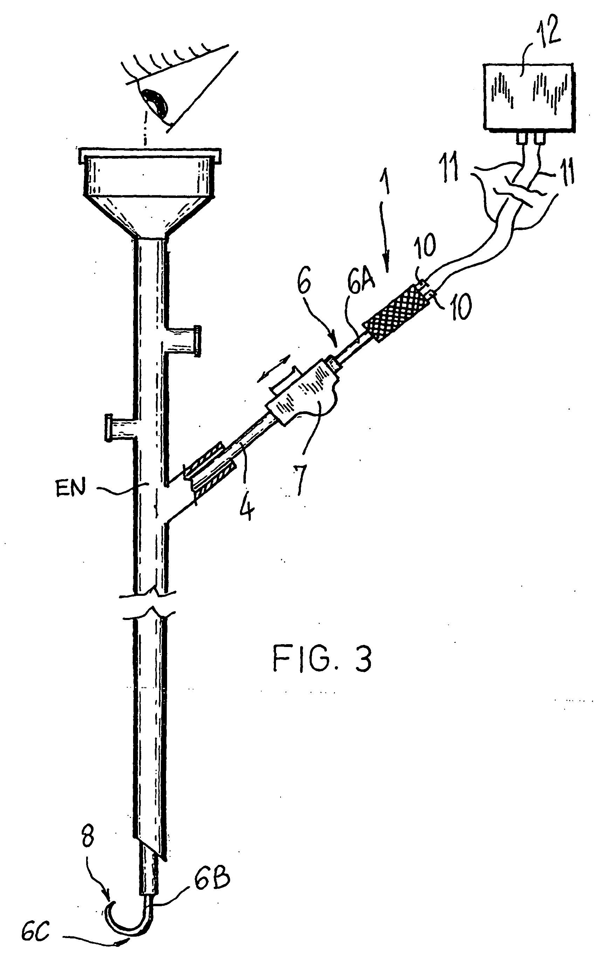

Figure 3 is an interrupted longitudinal-section view of an endoscope inside which the medical device of Figure 2, in a configuration of use is inserted.

### FORMS OF EMBODIMENT OF THE INVENTION

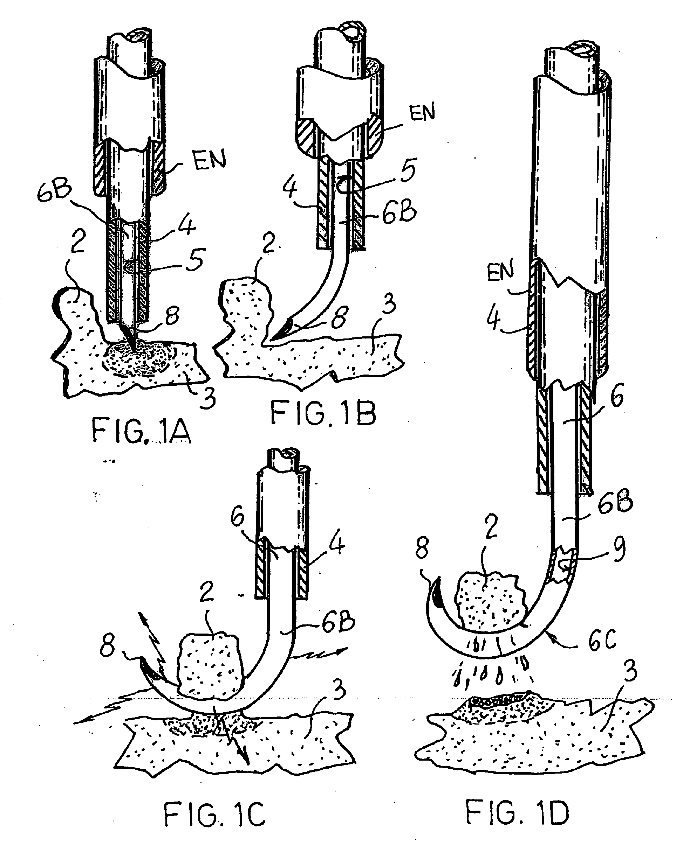

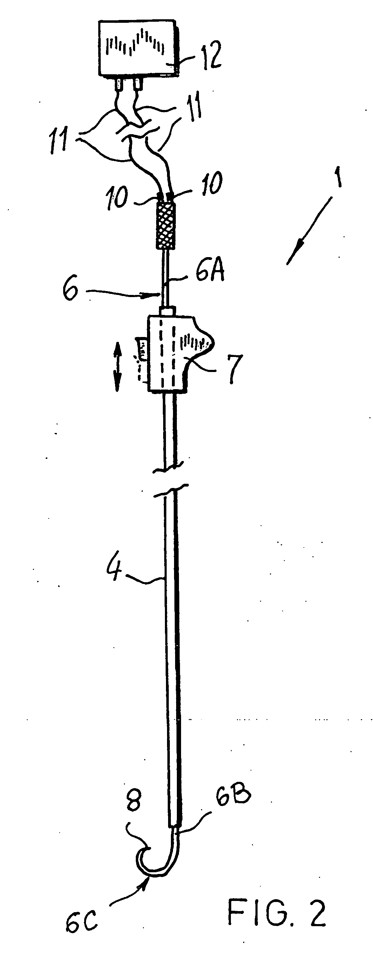

With reference to Figure 2, 1 indicates a medical device, suitable for cutting portions or excrescences 2 of human and/or animal tissues 3.

The medical device 1 comprises a flexible external sheath 4, through which a cavity 5 extends longitudinally in which can be inserted and moved by sliding a cannula 6, which is also flexible, and which is equipped with a handle 7 to allow the cannula 6 to be maneuvered and to slide the latter inside the external sheath 4 in a controlled manner.

The cannula 6 defines a proximal extremity 6A and an opposing distal extremity 6B which ends in a tip 8.

The cannula 6 is made of a flexible metallic material, specifically a material of the kind having a memory, i.e. that has the possibility to assume a configuration and to spontaneously return to said configuration after it is temporarily modified; also the cannula 6 is axially provided with a further cavity 9, which extends, on one end, up to the tip 8 and, on the other end, up to the proximal extremity 6A making the cannula 6 completely open for the administration of substances such as, for example, anesthetizing substances.

The distal extremity 6B of the cannula 6 defines an end segment which is shaped so as to form a loop 6C, designed to be placed near the excrescence 2 to be cut.

The shape of the loop 6C is maintained elastically straightened when the cannula 6 is inserted into the cavity 5 of the sheath 4, as can be seen in Figure 1a.

When the cannula 6 is pushed toward the excrescence 2 to be cut, as illustrate in Figures 1b, 1c, and 1d, the end segment of the distal extremity 6B, protruding from the sheath 4, progressively regains its shape of a loop 6C, shaped and memorized in advance by the metallic material with which the cannula 6 is made of: this material comprises, for example, Nitinol.

The proximal extremity 6A of the cannula 6 is equipped with electrodes 10 (or with a connector) that can be connected by means of their respective cables 11, to an electric current generator 12 which, when the medical device 1 is in a suitable position to begin a cut, is activated to heat the cannula 6 to a temperature that will allow it, or more precisely will allow the loop 6C, to cut an excrescences 2 that protrudes from the tissue 3.

After the cut is completed, the doctor modifies the intensity of the electric current supplied by the electric current generator 12 and, by maneuvering the cannula 6 through the proximal extremity 6A, places the loop 6C, in contact with the cut blood vessels, thereby cauterizing them.

In order to use the medical device the sheath 4 and the cannula 6, which is inserted in its longitudinal cavity 5, are both positioned, by means of an endoscope "EN" that was placed in advance in an endocavity where a cut is required, so that the distal extremity 6B is located near an excrescence 2, or another portion of tissue, that is to be cut.

The doctor then uses the handle 7, causing the cannula 6 to slide into the cavity 5 of the sheath 4, pushing the tip 8 out of the sheath 4 for a first, short segment, so that the cannula 6 maintains substantially straight, or with a very limited curvature, its distal extremity 6B.

By keeping temporarily stationary this configuration between the sheath 4 and the cannula 6, the doctor introduces the tip 8 into the tissue 3, in an area surrounding the excrescence 2 to be cut and injects through the further cavity 9 and the tip 8 an anesthetizing substance, connecting, for example, the proximal extremity 6A of the cannula 6 to a syringe "S" that has been previuosly filled with this substance: in this way, the doctor administers local anesthesia.

Subsequently the doctor slightly pulls the sheath 4 toward the proximal extremity 6A, causing it to slide along the cannula 6 and uncovering the end segment of the distal extremity 6B. Due to the memory of the metal (Nitinol) of which the cannula 6 is made, this distal extremity spontaneously regains the shape of a loop 6C which was given in advance to the distal extremity 6B.

In a subsequent step, the doctor places the loop 6C near the excrescence 2 to be cut and activates the electric current generator 12, causing the cannula 6 to be heated up to a temperature that allows, upon placing the loop 6C in contact with the excrescence 2, the latter to be cut.

Subsequently the doctor adjusts the intensity of the current to a suitable value and, by maneuvering the cannula 6 and its loop 6C, completes the operation by coagulating and cauterizing the cut blood vessels, by means of placing the loop 6C repeatedly in contact with them.

## Claims

1. Medical device (1) suitable for cutting and/or coagulating portions of human and animal tissues (2, 3), comprising sheath means (4); cutting means slidingly insertable into said sheath means and defining a proximal extremity (6A) and an opposing distal extremity (6B), said cutting means comprising cannula means (6) having a longitudinal passage (9) and administration means for administration of substances from said distal extremity (6B), said cannula means having a peripheral metallic heatable wall **characterized in that** said wall has a proximal extremity including connecting means (10,11) for connecting said proximal extremity to a source of heating energy (12), said longitudinal passage means (9) having both said distal and proximal extremities (6A, 68) open for the administration of substances.

2. Medical device (1) according to claim 1, wherein said cannula means includes: a long thin cylindrical metallic body (6) made of heatable material and defining said peripheral heatable wall; shaped means (6C) partially and/or completely wrappeable around said portions (2, 3), located at said distal extremity (6B); connecting means (10, 11) for connecting said proximal extremity (6A) to said source (12) of heating energy.

3. Medical device (1) according to claim 2, wherein said heatable material is a flexible metallic material.

4. Medical device (1) according to claim 3, wherein said flexible metallic material includes Nitinol.

5. Medical device (1) according to claim 1, wherein said longitudinal passage means comprises a conduit (9) that passes through said long thin metallic body (6) and that has opposing extremities defining corresponding apertures that lead, respectively, to said distal extremity (6B) and to said proximal extremity (6A).

6. Medical device (1) according to claim 2, wherein said shaped means comprises a distal segment of said long thin metallic body (6), partially curved upon itself so that to shape a loop (6C).

7. Medical device (1) according to claim 2, wherein said shaped means includes a distal segment of said long, thin metallic body (6), completely curved upon itself so that to shape a substantially closed ring (6C).

8. Medical device (1) according to claim 1, wherein said administration means includes a tip (8) that extends from said distal extremity (6B), said tip (8) having a longitudinally passage (9) which extends from said conduit and is designed to be introduced into a portion of tissue (2, 3) into which must be administered a substance.

## Patentansprüche

1. Medizinische Vorrichtung (1), die zum Schneiden und/oder Koagulieren von Teilen menschlichen und tierischen Gewebes (2, 3) geeignet ist, mit einer Ummantelung (4); einer Schneideinrichtung, die gleitend in die genannte Ummantelung einführbar ist und ein proximales Ende (6A) sowie ein diesem gegenüber angeordnetes distales Ende (6B) definiert, wobei die genannte Schneideinrichtung eine Kanüle (6) umfasst, die einen longitudinalen Durchgang (9) hat, sowie an dem genannten distalen Ende (6B) eine Appllkationseinrichtung zum Applizieren von Substanzen hat, wobei die Kanüle eine äußere, metallische heizbare Wandung hat, **dadurch gekennzeichnet, dass** diese Wandung ein proximales Ende mit einer Anschiusseinrichtung (16, 11) zur Verbindung des genannten proximalen Endes mit einer Quelle für Heizenergie hat, wobei der longitudinale Durchgang (9) sowohl an seinem genannten distalen Ende als auch an seinem genannten proximalen Ende (6A, 6B) für die Substanzappllkation offen ist.

2. Medizinische Vorrichtung (1) nach Anspruch 1, wobei die Kanüle folgendes umfasst: einen gestreckten, dünnen zylindrischen Metallkörper (6), der aus einem heizfähigen Material gefertigt ist und die genannte äußerte, heizbare Wand bildet; ein vorgeformtes Element (6C), das teilweise oder vollständig um die genannten Teile (2, 3) herum legbar ist und an dem genannten distalen Ende (6B angeordnet ist; eine Anschlusseinrichtung (10, 11) zur Verbindung des genannten proximalen Endes (6A) mit der genannten hleizenergiequelle (12).

3. Medizinische Vorrichtung (1) nach Anspruch 2, wobei das genannte heizbare Material ein flexibles metallisches Material ist.

4. Medizinische Vorrichtung (1) nach Anspruch 3, wobei das flexible metallische Material Nitinol umfasst.

5. Medizinische Vorrichtung (1) nach Anspruch 1, wobei der longitudinale Durchgang eine Rohrleitung (9) umfasst, die durch den genannten dünnen, metallischen Körper (6) hindurch tritt und einander gegenüberliegende Enden hat, die entsprechende Öffnungen beranden, welche jeweils zu dem genannten distalen Ende (6B) beziehungsweise dem genannten proximalen Ende (6A) führen.

6. Medizinische Vorrichtung (1) nach Anspruch 2, wobei das genannte vorgeformte Element ein distales Segment des genannten lang gestreckten, dünnen metallischen Körpers (6) umfasst, das in sich selbst teilweise so gekrümmt ist, dass es eine Schleife (6C) formt.

7. Medizinische Vorrichtung (1) nach Anspruch 2, wobei das genannte vorgeformte Element ein distales Segment des genannten langen, dünnen metallischen Körpers (6) umfasst, das in sich vallständig gekrümmt ist, so dass es einen im wesentlichen geschlossenen Ring (6C) bildet.

8. Medizinische Vorrichtung nach Anspruch 1, wobei die genannte Applikationseinrichtung eine Spitze (8) umfasst, die von dem genannten distalen Ende (6B) ausgeht und wobei die genannte Spitze (8) einen longitudinalen Durchgang (9) hat, der von der genannten Rohrleitung ausgeht und so ausgebildet ist, dass sie in einen Teil des Gewebes (2, 3) einführbar ist, in das eine Substanz eingebracht werden muss.

## Revendications

1. Dispositif médical (1) approprié pour couper et/ou coaguler des parties de tissus humains et animaux (2, 3), comprenant des moyens formant gaine (4) ; des moyens de coupe qui peuvent être insérés en glissant dans lesdits moyens formant gaine et qui définissent une extrémité proximale (6A) et une extrémité distale (6B) à l'opposé, lesdits moyens de coupe comprenant des moyens formant canule (6) qui présentent un passage longitudinal (9) et des moyens d'administration destiné à administrer des substances à la sortie de ladite extrémité distale (6B), lesdits moyens formant canule ayant une paroi métallique périphérique pouvant être chauffée ; **caractérisé en ce que** ladite paroi a une extrémité proximale qui comprend des moyens de connexion (10, 11) servant à connecter ladite extrémité proximale à une source d'énergie de chauffage (12), lesdits moyens formant passage longitudinal (9) ayant lesdites extrémités distale et proximale toutes deux ouvertes pour l'administration de substances.

2. Dispositif médical (1) selon la revendication 1, dans lequel lesdits moyens formant canule comprennent un corps métallique cylindrique long et mince (6) fait d'une matière pouvant être chauffée et qui définit ladite paroi périphérique pouvant être chauffée ; des moyens conformés (6C) qui peuvent s'enrouler partiellement ou complètement autour desdites parties (2, 3), qui sont situés à ladite extrémité distale (6B); les moyens de connexion (10, 11) servant à connecter ladite extrémité proximale (6A) à ladite source d'énergie de chauffage (12).

3. Dispositif médical (1) selon la revendication 2, dans lequel ladite matière pouvant être chauffée est une matière métallique flexible.

4. Dispositif médical (1) selon la revendication 3, dans lequel ladite matière métallique flexible inclut le Nitinol.

5. Dispositif médical (1) selon la revendication 1, dans lequel lesdits moyens formant passage longitudinal comprennent un conduit (9) qui passe dans ledit corps métallique long et mince (6) et qui a des extrémités opposées définissant des ouvertures correspondantes qui conduisent respectivement à ladite extrémité distale (6B) et à ladite extrémité proximale (6A).

6. Dispositif médical (1) selon la revendication 2, dans lequel lesdits moyens conformés comprennent un segment distal dudit corps métallique long et mince (6), partiellement recourbé sur lui-même de manière à former une boucle (6C).

7. Dispositif médical (1) selon la revendication 2, dans lequel lesdits moyens conformés comprennent un segment distal dudit corps métallique long et mince (6) complètement recourbé sur lui-même, de manière à former un anneaux sensiblement fermé (6C).

8. Dispositif médical (1) selon la revendication 1, dans lequel lesdits moyens d'administration comprennent une pointe (8) qui part de ladite extrémité distale (6B), ladite pointe (8) présentant un passage longitudinal (9) qui s'étend à partir dudit conduit et qui est conçu pour être introduit dans une partie de tissu (2, 3) dans laquelle une substance doit être administrée.
